# EUROPEAN PATENT APPLICATION

(11) **EP 0 612 510 A2**
(43) Date of publication of application: **31.08.1994**
(21) Application number: 94670001.0
(22) Date of filing: 25.01.1994
(51) Int. Cl.: A61F 9/06

(54) **Positioning control device for a hand welder helmet visor**

(30) Priority: 25.01.1993 PT 10118793
(71) Applicant: Dos Anjos Lopes, José Manuel, P-2700 Amadora (PT)
(72) Inventor: Dos Anjos Lopes, José Manuel, P-2700 Amadora (PT)
(74) Representative: Ferreira, Maria Silvina

(57) **Abstract**

The positioning control device of a hand welder helmet visor (9) comprises a control being fitted on the welding tool handle (2) and manageable by its holding user's hand, and insures that the welding tool only will be put in operation if the visor is brought down.

One of its embodiments includes lever means (1), a flexible wire (4) slidable inside a sheath (5), a spring (6) fitted on the hinge of the visor (8) and blocking means (3) which hinders the welding tool operation whenever the visor is brought up.

It will be also possible to use a pneumatical, hydraulic, electrical and electromagnetical radiation transmission between the tool handle and the visor.

The technical field of the invention embraces the operator protection helmets for welding operations and similar ones.

## Description

The invention refers to a positioning control device of a hand welder protection helmet visor manageable according to its user's conveniences or for operators doing similar workshop operations. Said device is conditioned in order to insure that the welding tool only will may be put in operation if the visor is brought down; on the other hand, its control is fitted near the welding tool handle so that it may be operated by the welder without needing to withdraw one of his hands from the piece to be welded as it has been necessary up till now.

Some workshop operations must be practiced forcing their operators to work under the protection by a helmet for their eyes against the harmful bright radiation action or against the strike of solid particles or dust being thrown at high speed or fumes, for instance, welding, air jet cleaning operations, emery grinding, inter alia, the helmet being fastened on their heads and having a visor that must be brought down whenever the tool is in operation and may be brought up when the operation is interrupted and the operator wishes to relax the eyes or to examine in detail the piece being treated. On the presently existing and known helmet models for protecting a welder, the positioning of the visor thereof is directly accomplished by the operator using one of the hands and therefore having only a hand available for the work execution, or the bringing up and the bringing down of visors is obtained by means of a liquid crystal control operating independently of the operator's intention. On the other side, the operator can bring up the visor, even if the physical integrity of his eyeballs may be exposed to danger, and can also begin the work without the visor duly brought down.

It should be emphasized that this fact has a very special interest just in the case of welding operations where the visor is meant for absorbing the ultraviolet radiation which are emitted during the welding process and for preventing damages to the welder's eyeballs (namely, the retina separation) ; however, the visor also absorbs strongly the visible radiations and renders extremely difficult the vision of the piece to be welded when the operation of the welding device is cut off. Usually, the bringing down and the bringing up of the welding helmet visors are accomplished by the welder with one of his hands while holding the flame torch or the welding pliers with the other. The consequences of such a fact are as follows: the over-all welding process is much slower; the danger of the arc sparking before the visor being brought up arises; and these consequences are even worse whenever the welder needs to hold or roll the piece being weld.

The object of the present invention is the elimination of the need that the operator will bring down or will bring up the protecting helmet visor directly with one of his hands, while only the other hand will remain available for making the welding work, making possible the obtainment of the resultant advantages consisting of a better security and comfort for the operator and a greater output of the welding operation.

Such an object is achieved, according to the present invention, by means of a control device having the above mentioned features and which may be of mechanical nature, or pneumatical, hydraulic, electric (comprising a servomechanism and a small electric motor) or electronic (comprising a servomechanism and a small radio-controlled motor) type.

The electronic solutions are the handiest ones since they avoid any physical contact - tubes, cables or wires - between the welding device and the helmet. They consist essentally of a monocontrol miniature transistorized, lightweight and low consumption set. A radio-receptor fitted inside or outside the helmet picks up a radio signal which is emitted by a transmitter placed on the handle of the welding pliers or flame torch and converts it into the necessary electric current for bringing up or bringing down the visor by means of an immediate response servomechanism or a less immediate response motor.

The advantages offered by this invention are essentially that the welder's security is always insured, a greater comfort for the welder is achieved, a better output of the welding operations are obtained and the welder may always have both hands at his disposition during all the welding operations. However, due to its greater simplicity and constructive easiness, and low cost, an embodiment of the present invention based on a purely mechanical solution to be applied to electrical welding pliers, will be subsequently described with reference to the enclosed drawing wherein
Figure 1 shows a schematical representation of a perspective view of a mechanical device for controlling the positioning of a welding helmet visor according to the present invention;
Figure 2 shows a schematical representation of an enlarged perspective view of the visor fastened on the helmet with a part of the side wall removed; and
Figure 3 shows a schematical representation of an enlarged perspective view of the welding tool handle having the device of Figure 1 duly applied.

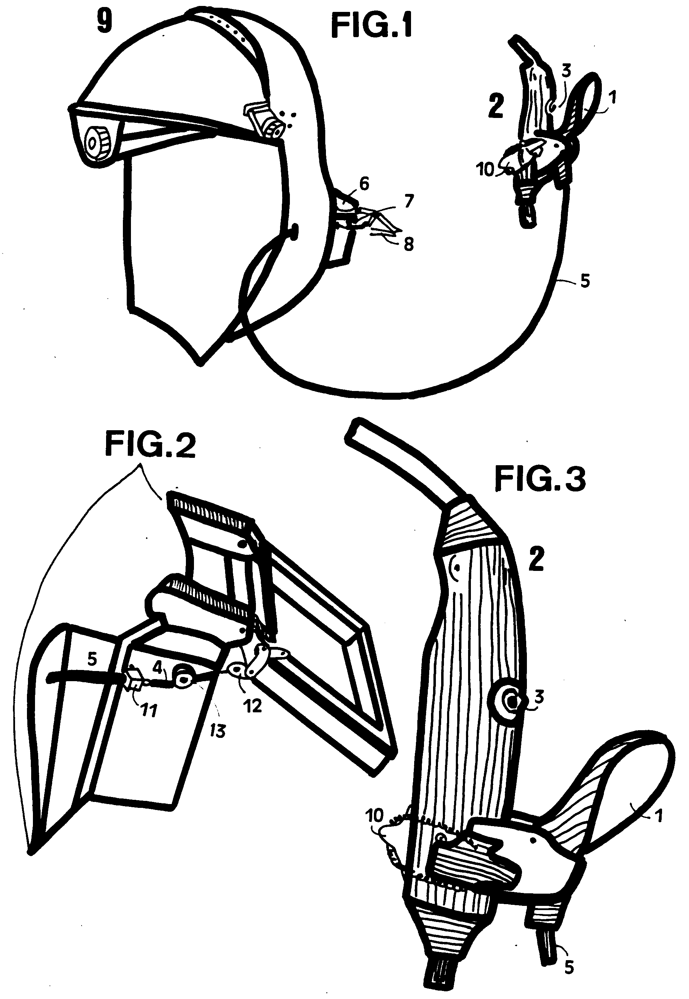

As Figure 1 clearly shows, the embodiment of the mechanical device for controlling the visor position comprises essentially a lever mechanism (1) fitted on the handle (2) of the electrode-holder pliers of the welding tool by means of a self-adjustable spring clamp (10); a slidable wire (4) inside a flexible sheath (5) interconnecting the lever mechanism (1) and a spring (6) fitted on the hinge (7) of the helmet (9) visor (8); a blocking device (3) which interrupts the electric power feeding to the welding pliers whenever the visor is in a brought up position, in this case, a switch. The lever mechanism may assume two stable positions, each one of them being selectable by the operator at his conveniences: one of them corresponds to the brought up position of the visor (6) and the other position corresponds to the brought down position of the visor. Both these positions are transmitted by means of the wire (4) sliding within the sheath (5). The sheath (5) is fixed on the helmet wall by means of an adjustable screw (11) while the slidable wire, after passing through a guiding roller (13), actuates a visor positioning support (12) and operates against the spring (6) of the visor (8) hinge (7).

The operating way of the device is very easy to understand. When the welder presses the lever (1) against the handle (2) of the welding pliers, the lever system pulls the wire (4) and, as a consequence, the visor is brought down through the spring (6) action and therefore protects the operator's eyes. Only after this action, the lever (1) is able to actuate the switch button (3) and the electric current is fed to the welding tool; in this way, the complete security of the welder is insured, since the arc only may be established after the visor having assumed the brought down position. When the welder completes the welding work or wishes to look at the welded piece, he interrupts the pressing upon the lever (1), this releases the switch button (3) cutting the feeding current to the welding tool e only afterwards the visor is brought up by the action of the spring (6).

If a flame torch is used, the difference will be that the blocking means (3) is a blocking cut-off valve interrupting the gas flow.

## Claims

1. A positioning control device of a hand welder helmet visor, manageable according to its user's conveniences, characterized in that its control is fitted on the welding tool handle so that it may be operated by the user's holding hand and conditions the operation feasibility of the tool only to the case where the visor is brought down.

2. The positioning control device of a hand welder helmet visor, according to claim 1, characterized in that said device includes:
(a) lever means (1) fitted on the welding tool handle (2) being able to assume one of two stable positions, selected by the user according to his conveniences, corresponding respectively to the brought down or to the brought up position of the visor, and which acts as the control of the device;
(b) a flexible wire (4) slidably encased within a sheath (5);
(c) a spring (6) fitted on the hinge (7) of the helmet visor (8); and
(d) blocking means (3) which hinders the welding tool operation whenever the visor is brought up.

3. The positioning control device of a hand welder helmet visor, according to claim 2, characterized in that said blocking means (3) consists of an electric current switch, when the welding tool is electrical welding pliers.

4. The positioning control device of a hand welder helmet visor, according to claim 2, characterized in that said blocking means (3) consists of a shut-off valve fitted on the gas feeding tubing, when the welding tool is a flame torch.

5. The positioning control device of a hand welder helmet visor, according to anyone of preceding claims, characterized in that said visor (8) is a visor which is either an usually brought up one or an usually brought down visor.

6. The positioning control device of a hand welder helmet visor, according to claim 1, characterized in that said control has a pneumatical, hydraulic, electric or electromagnetic radiation transmission system between the handle and the visor and uses the corresponding fittings.
